# EUROPEAN PATENT APPLICATION

(11) **EP 2 441 732 A1**
(43) Date of publication of application: **18.04.2012**
(21) Application number: 09844707.1
(22) Date of filing: 29.10.2009
(51) Int. Cl.: C01B 31/06, B82B 1/00, B82B 3/00, B01J 3/06, A61P 35/00

(54) **CARBON-BEARING NSP3 NANOPARTICLE AND A METHOD FOR THE PRODUCTION THEREOF**

(30) Priority: 15.05.2009 RU 2009118268
(71) Applicant: Obshchestvo S Ogranichennoy Otvetstvennostyu "Nano-s", Moscow 117574 (RU)
(72) Inventor: STAVRI, Yanev Stavrev, 1784 Sofia (BG)
(74) Representative: Dennemeyer & Associates S.A.
(86) International application number: PCT/RU2009/000588
(87) International publication number: WO 2010/131992

(57) **Abstract**

The invention relates to nanotechnology. The carbon-bearing nanoparticle consists of a cubical carbon monocrystal nucleus, the size of which is equal to or less than 4 nm, and a monocrystalline carbon shell having an SP³ structure and a thickness ranging from 0.19 to 0.2 nm. The method for producing carbon-bearing NSP³ nanoparticles involves exploding a charge of blasting material having a negative oxygen balance. The charge is preliminary surrounded by ice with a temperature less than minus 25°C. The ratio of the mass of the ice to the mass of the explosive charge is of 1:4-6. The suspension of carbon-bearing nanoparticles produced as a result of explosion is chemically purified. The thus produced suspension is disaggregated by being repeatedly frozen to a temperature lower than the liquid hydrogen boiling point. Once the suspension has been disaggregated, it is exposed to the action of ultrasonic waves with a frequency of 18-27 Hz for 5-18 minutes. The blasting material consists of a trinitrotoluene and cyclonite mixture and is in the form a cylinder-shaped plastic mixture with a ratio of the cylinder diameter to the height thereof of 1:6. The method for chemically purifying the suspension involves removing metal impurities from the suspension by heating it for 5-7 hours in a 10-25% nitric acid solution.

## Description

The group of the inventions relates to nanotechnology, namely to the area of synthesis of nanoparticles and, in particular, to the synthesis of nanodiamond and its preservation in disaggregated state. The claimed engineering solutions can be used in creation of medicinal products, which include nanoparticles in their composition.

Nanodiamonds belong to the class of nanoparticles characterized by extreme instability, which is a complication for their further usage as from the very moment of production the nanodiamonds aggregate and form agglomerates of particles. There is a method of *production of detonation nanodiamond,* which consists of treatment of diamond load by mixtures of acids under high temperature. The treatment is carried out with the use of aqueous solutions of nitric acid under pressure,
which ensures the process flow in a liquid phase at two stages: during the first stage - by aqueous solutions of 50-99 % nitric acid at 80 - 180 °C, and during the second stage - 10 - 40 % nitric acid at 220 - 280 °C (patent of invention in RF No. 2109683). The method currently in use produces a composition material, which consists of two components: diamond monocrystallic nucleus of 1 - 120 nm with numerous surface defects,
and and sandwich shell, which consists of amino groups (hydroxylic, carbonylic, carboxylic, quinonic, clearl carbonic), which makes it impossible to use the obtained nanoparticles, due to the fact that the nanoparticles need the modification of the shell by chemical methods, and sometimes this modification makes them toxic. Currently there is a *method of the diamond clearing,* which consists of mixing the diamond-bearing
raw material with 5-20 % nitric acid solution at 50 - 100 °C, the residue at 60 - 90 % RH is treated with oxidative sulphuric-nitric or nitro-oleumic mixture in a flow reactor (inventor's certificate in USSR No. 1770272). The method in use increases the speed of the process of the separation of the contaminants (microparticles) but it does not support synthesis of nanomaterials.

*Nanodiamond* containing carbon, hydrogen, nitrogen and oxygen is known. Herewith the carbon is the part of nanodiamond as a diamond cubic modification in X-ray amorphous phase in the ratio of (82-95):(18-5) % wt. w/w of carbon respectively, and *the method of its synthesis,* which involves confined detonation of carbon-bearing explosive material with negative oxygen balance in carbon-inert gas environment in condensed phase, and subsequent chemical purification with detonation of the explosive material placed in the shell of condensed phase containing reducing agent, with the quantitative ratio of the weight of the reducing agent in the condensed phase to the weight of the carbon-bearing explosive material of not less than 0.01:1.

The chemical purification is provided by treatment of the detonation products by aqueous nitric acid with oxygen contained in compressed air at 200-280 °C and 5-15 MPa (patent of invention in RF No. 2348580).

However the method currently in use results in the synthesis of aggregated diamond nanoparticles and it can not be used for medical prposes due to their high toxicity and besides the known nanodiamonds have disorganized structure and wide range of cross-cut diameters, which complicate their usage.

The closest to the mentioned group of inventions in terms of technical essence is the method of synthesis of *biologically active nanodiamonds by detonation,* which involves synthesis of special mixed explosive materials (EM) by detonation (explosion). These materials contain the excess of carbon atoms (as compared to the oxidizing agent) in the molecule of the EM in nonoxidizing environment (gaseous, liquid, solid). The diamond load synthesized after the explosion contains 30-60 %wt. sized 1-120 nm (mean size is 4-6 nm) and is subjected to the chemical purification involving oxidation of non-diamond carbon (35-65 %wt.) and dilution of metallic admixtures (2-5 %wt.) in aqueous nitric acid at high temperatures (240-260 °C) and pressure (up to 100 atm.). The diamond microcrystalls synthesized in extremely non-stationary conditions in the front of detonation wave during very short lapse of time (less than 10⁻⁶ s) have numerous surface defects. Thereby the surface carbon atoms of diamond crystals have no time to stabilize its electron shell in a standard way, by closing of its
unpaired electrons with respective connections inside the crystal with internal carbon atoms. Therefore active electron shell of the surface carbon atoms in the diamond crystals is stabilized by forming an "edging" of different hydrogen-bearing groups of oxygen and hydrogen contained in the molecules of the initial EM. Thus, the nanodiamonds crystals with a chemically passive nucleus of classic
cubic diamond of spheric or oval shape without side cutters, at the same time have a considerably reactive (though weak) surface "edging" of functional groups that are non-hazardous for a living organism (oxy-, carboxy-, carbonylic etc.) (description of invention for the patent in RF No. 2203068). During the synthesis of nanodiamonds (ND) by the known method, the detonation physics
leads to formation of heterogenetic ND and agglomerates of ND with the size ranging from hundreds of nanometers to few microns. The presence of microparticles in the obtained substance is almost insuperable obstacle to its usage for medical purposes. The claimed group of technical solutions is aimed at creation of carbon-bearing nanoparticles and route of their synthesis that allows to synthesize and preserve the
desaggregated state of nanoparticles with minimal toxicity that allows the use of the obtained nanodiamond particles for medical purposes. Technically, the use of the claimed group of inventions results in elimination of the free radical properties and adjustment of nanoparticles sizes. The target is achieved by the fact that carbon-bearing nanoparticle is composed by carbon
cubic monocrystallic nucleus sized not more than 4 nm and monocrystalline carbon shell with an SP³ structure, with thickness ranging from 0.19 to 0.2 nm. The route of synthesis of carbon-bearing NSP³ nanoparticles, ***according to the claimed technical solution,*** involves *exploding a charge* of explosive material with a negative oxygen balance. The charge is pre-packed in ice cooled below -25°C. The ratio of the weight of the ice to the weight of the explosive charge is of 1:4-6. The *suspension of carbon-bearing nanoparticles* synthesized as a result of explosion is *chemically purified.* The suspension obtained in this way is *disaggregated* by being repeatedly frozen below liquid hydrogen boiling point. Once the suspension has been *disaggregated,* it is sonicated at 18-27 Hz for 5-18 minutes. The number of sequential freezing of the suspension should be not less than 3.

The explosive material contains trinitrotoluene and cyclonite mixture and is in the form a cylinder-shaped plastic mixture with cylinder diameter to height ratio of 1:6. The method of chemical *purification* the suspension involves removal of metal impurities from the suspension by heating in 10-25% nitric acid solution for 5-7 hours. The claimed nanoparticles have limited size of 2-4 nm and sharp-edged homogeneous structure. The base of the nanoparticle contains carbon cubic monocrystallic nuclear of high density. The nucleus is covered by a monolayer shell consisting of monocrystallic carbon with SP³ structure and the thickness of 0.197 nm.

The claimed method is performed as follows.

The EM with negative oxygen balance, which is trinitrotoluene (TNT) and cyclonite (RDX) mixture, is prepared for the explosion. Oxygen balance is the excessive, sufficient or insufficient quantity of oxygen in the explosive material as compared with the amount required for full oxidation of oxygen, hydrogen and other elements oxidized during the explosion. The carriers of oxygen in the EM are usually salts of nitric acid: ammonia nitrate, sodium nitrate, potassium nitrate etc. Oxygen balance can have a zero, positive or negative value. Zero oxygen balance in the EM is assumed as that with oxygen in the amount sufficient for full oxidation of all fuel pellets forming part of the EM, such as carbon, hydrogen or solid combustible additives. If the amount of oxygen is insufficient for full oxidation of combustible elements oxygen, the balance is considered negative. Trinitrotoluene is a yellowish crystal substance with melting point of 80.35 °C (melts in very hot water). The explosive conversion energy is 1010 kkal/kg. Velocity of detonation wave is 6700 - 7000 m/s (density: 1.6 g/cm³). Heat of explosion is 4228 kJ/kg. Brisance index by Hess is 16 mm. Brisance index by Cast is 3.9 mm.

Cyclonite is one of the strongest and high-order explosives. It appears as white crystal substance with specific gravity of 1.8, and melting point of 205 °C with decomposition.

The mixture for the explosion is prepared in proportional ratio: trinitrotoluene - 50-70 % and cyclonite - 30-50 %. The EM is shaped as a cylinder with a ratio of the cylinder diameter to its height of 1:6. For the detonation the EM (the charge) is put into the explosion chamber with the volume of 2-3 m³ and afterwards the charge is cooled by surrounding it with ice cooled below -25 °C.

The ratio of the weight of the ice to the weight of the explosive charge is of 1:4-6. The best ratio is 1:5. Explosion of the charge is performed immediately after surrounding it with ice using electrodetonator placed at the end of the charge inside of it. The cylindrical shape of the charge results in radial-axial expansion of detonation wave front. Formation of nanodiamond particles occurs in detonation wave in the zone of chemical reaction. The diamond is the phase of high pressure. During the EM detonation the optimum conditions for its formation are formed in the detonation wave. The diamond in the detonation wave is formed as a result of polymorphic transformation of amorphous carbon. Explosion of a charge of explosive material leads to formation of aqueous suspension of diamond load that is poured from the explosive chamber into the receiving tank 3 minutes after the explosion. The suspension of diamond load is filtered to separate gross and fine impurities, subject to magnetic separation and centrifuge till obtaining a dense and easily detachable cake. The wet diamond load is further transferred to the stage of chemical purification where it is placed into 60-% HNO₃ (nitric acid), subjected to hard dispersion, filtered up to hard-to-remove impurities and transferred by flow-control pumps to the system of flowing combined-profiled titanic reactors. The process temperature is 230-240 °C, the pressure is 8-10 MPa. The holding time of reaction weight in the heat zone is 30-40 min. The diamond load is purified in the described conditions from non-diamond carbon and non-combustible impurities consisting in the form of complex salts. After the separation of organic impurities and washing of nanoparticles, the suspension is treated by 15 % solution of nitric acid at 60 °C during 6 hours to eliminate metallic impurities. After chemical purification of carbon-bearing suspension, disaggregation of the nanoparticles contained in the suspension is performed. The suspension is frozen three times to liquid nitrogen boiling point with intermediate defrosting up to ambient temperature. During the freezing the pressure is increased up to 2500 kg/mm² and the process of nanoparticles aggregates decomposition is initiated. After freezing for three times the suspension is subjected to hard ultrasonic processing by the dispergator during 5-18 minutes at 18-27 Hz. Nanoparticles processed in this way keep stability to formation of stable (primary) agglomerates during 6 months.

The formed unstable (secondary) agglomerates are easily decomposed during the processing by light ultrasonic impulse.

Nanoparticles obtained by the claimed method have a standard size of 2-4 nm, total surface area >420 m²/g, chemical purity 99.99 %, no metallic impurities, and abnormally high absorption characteristics (1 to 10 µg-Eq/m²). The combination of the described properties creates opportunities for their use for medical purposes. In particular, the test of NSP³-nanoparticles effect on cell proliferation was performed. After addition of the suspension containing NSP³-nanoparticles to the cell structure the process of nanoparticles attachment to the cell surface is initiated. NSP³-nanoparticles were firmly attached to the cell surface during the first 15 minutes of their coexistence and were attached
during all the time of observation. 3 hours later the bond was very strong and expanded and 6 hours later the cells kept the attached nanoparticles. The observations show that NSP³-particles can be widely attached to the cell membrane (the same results were achieved in observations using an optic microscope and HeLa cells) and as the particles do not inhibit the cell proliferation, the cells can be used in combination with
the known biomaterials, which can improve their ability to attach and expand on the cell. The test of possible toxic effect of NSP³-nanoparticles was conducted on animal cells with two permanent cell lines: Vero (kidney cells of Cercopithecus aethiops) and HeLa (epileptoid cervical carcinoma, human cells). Metabolic activity of the cells was monitored by MTT assay. On average, NSP³-particles have a toxic effect on HeLa cells in 30 % of the cases and only at high concentrations (165 and 1650 µg/ml) and only after long-term exposal. The use of the claimed method results in obtaining suspension containing NSP³-nanoparticles with minimum toxic level, which allows to use them for medical treatment, in particular, during the treatment of oncological diseases.

## Claims

1. Carbon-bearing NSP³ nanoparticle, ***characterized by*** cubical carbon monocrystal nucleus, the size of which is equal to or less than 4 nm, and a monocrystalline carbon shell having an SP³ structure and a thickness ranging from 0.19 to 0.2 nm.

2. The route of synthesis of carbon-bearing NSP³ nanoparticles, ***characterized by*** explosion of a charge of explosive material with a negative oxygen balance, the charge is pre-packed in ice cooled below -25°C, the ratio of the weight of the ice to the weight of the explosive charge is of (4-6) : 1, the suspension of carbon-bearing nanoparticles synthesized as a result of explosion is chemically purified, the suspension obtained in this way is disaggregated by being repeatedly frozen below liquid hydrogen boiling point.

3. The route of synthesis of carbon-bearing nanoparticles described in claim 2, ***characterized by*** sonication of the suspension at 18-27 Hz for 5-18 minutes after *disaggregation.*

4. The route of synthesis of carbon-bearing nanoparticles described in claim 2, ***characterized by*** consequential freezing of the suspension not less than 3 times during *disaggregation.*

5. The route of synthesis of carbon-bearing nanoparticles described in claim 2, ***characterized by*** the composition of the explosive material, containing trinitrotoluene and cyclonite mixture, which is a cylinder-shaped plastic mixture with a ratio of the cylinder diameter to its height of 1:6.

6. The route of synthesis of carbon-bearing nanoparticles described in point 2, ***characterized by*** the *chemical purification* of the suspension performed by heating during 5-7 hours in 10-20 % nitric acid solution, which ensures elimination of metallic impurities from the suspension.
